# EUROPEAN PATENT APPLICATION

(11) **EP 2 324 766 A2**
(43) Date of publication of application: **25.05.2011**
(21) Application number: 10251801.6
(22) Date of filing: 15.10.2010
(51) Int. Cl.: A61B 5/103

(54) **Micro-insult test and use therefor**

(30) Priority: 16.10.2009 US 580819
(71) Applicant: Johnson & Johnson Consumer Companies, Inc., Skillman, NJ 08558 (US)
(72) Inventor: Bargo, Paulo R., Hamilton Square, NJ 08690 (US); Kollias, Nikiforos, Skillman, NJ 08558 (US); Seo, Inseok, Plainsboro, NJ 08536 (US)
(74) Representative: Kirsch, Susan Edith

(57) **Abstract**

The present invention relates to a method for testing human skin and other biological tissues, and the use thereof for testing the effects of ingredients and compositions on such tissues *in vivo.*

## Description

### FIELD OF THE INVENTION

The present invention relates to a method for testing human skin and other biological tissues, and the use thereof for testing the effects of ingredients and compositions on such tissues *in vivo.*

### BACKGROUND OF THE INVENTION

Testing cosmetic products on human skin has always been a vital component of both basic and applied research. There is always some concern with human skin testing, in that an adverse effect may result in a visible alteration of the skin, such as discoloration or scarring. For this reason, animal testing has typically preceded human skin testing. The development of alternative test systems for human skin is of increasing priority due to recent European Community regulations banning the use of animal testing for cosmetic ingredients.

Several methods have been developed to probe biological tissues, particularly skin (due to its easy accessibility), *in vivo* using macroscopic tools. For example, approved protocols for testing the effects of UV radiation on skin require exposing a 1 cm² area of skin to UV radiation. Prick tests for evaluation of sensitivity to allergens is also a common practice in allergy clinics.

Some microscopic techniques have been developed for the treatment of skin aging, i.e., reduction of wrinkles, to minimize the harm to epidermis and reduce the risk of side effects, complications, and downtime. Such techniques involve inducing an array of microscopic wounds on the skin surface that are rapidly re-epithelialized by the surrounding, undamaged tissue, sparing the epidermis. A laser with infrared wavelength is often used, and the radiation is absorbed by aqueous components inside the tissue with limited and controlled areas of photocoagulation that stimulate a therapeutic response deep in the dermis. This leads to increased production of neo-collagen and improvement in skin tone and texture.

It is known in the art to use a variety of macro and micro imaging techniques to evaluate the properties of human skin. However, application of microscopic wounds for the purpose of skin analysis, rather than treatment of macro features such as wrinkles, has prior to now not been done.

There is a continuing need for a human skin test that minimizes permanent, visible damage to the skin yet is predictive of the effect on the metabolic activity and the structural integrity of the skin. Applicants have now discovered an effective method of testing human skin that features the combination of applying one or more micro-insults to an area of human skin with monitoring such area using an imaging technique.

### SUMMARY OF THE INVENTION

The present invention is directed to a method for testing human skin, which comprises causing at least one micro-insult to an area of the human skin; and monitoring the area using an imaging technique.

In a second embodiment, the present invention provides a method for determining the effects of a topically applied composition to human skin, which comprises in sequence applying the composition to an area of the human skin; causing at least one micro-insult to the area; and monitoring the area using an imaging technique.

In a third embodiment, the present invention provides a method for determining the effects of a topically applied composition to human skin, which comprises in sequence causing at least one micro-insult to an area of the human skin; applying the composition to the area; and monitoring the area using an imagining technique.

### BRIEF DESCRIPTION OF THE DRAWINGS

FIGs. 1A and 1B are cross-polarized images of micro-wounds described in Example 1.
FIGs. 2A, 2B, 2C, 2D and 2E are confocal microscope images of the micro-wounds described in Example 1 after 30 minutes.
FIG. 2F shows the intensity depth profile from three different regions of one confocal microscopy image stack for a subject: treated area, surrounding area and untreated area, as described in Example 1.
FIGs. 3A, 3B, 3C and 3D are cross-polarized images of microscopic wounds described in Example 1 acquired with video microscopy at 30 minutes, 2 days, 4 days and 23 days after treatment.
FIG. 4 depicts the confocal microscopic images and depth intensity profiles for the microscopic wounds shown in FIGs. 3A, 3B, 3C and 3D.
FIGs. 5A, 5B and 5C are normalized depth intensity profiles of a 25 year old subject, a 53 year old subject and a 30 year old subject at different time points after treatment, as described in Example 1.
FIG. 6 is a spectrum profile of the solar simulator in comparison with the COLIPA standard, as described in Example 2.
FIGs. 7A and 7B are cross-polarized images of UV irradiated sites through optical fibers and circular slits, as described in Example 2.
FIG. 8 depicts cross-polarized microscopic (20 x- 400x magnification) images taken at different days after UV irradiation of a subject, as described in Example 2.
FIG. 9 shows cross-polarized microscopic images taken at days 0, 1 6 and 10 says after UV irradiation of a subject, as described in Example 2.
FIG 10A shows the cross-polarized images of a UV phototest performed using the method according to the invention and a standard COLIPA-recommended method, as described in Example 3.
FIG 10B shows cross-polarized images of a UV phototest performed performed using the method of the invention and a standard COLIPA-recommended method application of a sunscreen, as described in Example 3.

### DETAILED DESCRIPTION OF THE INVENTION

Although the invention has been primarily described as applicable to testing human skin, the methods and systems described herein may also be used for the testing of other human and animal biological tissues, such as hair, lips, oral mucosa, and internal organs (esophagus, arteries, intestines, liver, etc.), for example optionally with the use of optical fibers or laparoscopic/endoscopic techniques.

The invention provides a method for testing human skin, which comprises causing at least one micro-insult to an area of the human skin; and monitoring the area using an imaging technique.

In one embodiment, the method may be used for evaluating the effects of ingredients or compositions on human skin. For example, the method may be used for allergy testing or cosmetic product/drug efficacy testing. The effects of anti-acne products, anti-aging products, sunscreen products, or wound healing products, or the like, for instance, may be tested according to the invention.

Accordingly, the invention also provides a method for determining the effects of a topically applied composition to human skin, which comprises applying the composition to an area of the human skin; causing at least one micro-insult to the area; and monitoring the area using an imaging technique. Application of the composition may be done before or after the micro-insult is applied to the skin.

As used herein, the term "micro" means having a maximum dimension of less than about 1,000 microns, such as less than about 500 microns. For example, dimensions in the range of about 0.5 µm to about 5 µm are on the sub-cellular level, dimensions of about 5 µm to 50 µm are on the level of whole cells, and dimensions of about 50 µm to about 1,000 µm are on the level of multiple cells.

As used herein, the term "micro-insult" means a wound or injury of micro size. Such wound or injury may be caused for example by punctures, cuts, burns, irradiation, or exposure to allergens. Examples of micro-insults include, but are not limited to, laser micro-injury, UV micro-irradiation, microinjection, and microscopic puncture. Micro-insults in the form of exposure to allergens may be inflicted using capillary tubes or micro-needles to deliver the allergens. Micro-insults in the form of burns may be inflicted using microscopic heating probes or radio-frequency generators. Micro-insults in the form of punctures or cuts may be delivered using micro-needles.

The shape of the micro-insults is not critical. They are preferably disconnected and spaced far enough apart so that each can be monitored individually. Spacing between the micro-insults may be at least about 10 um, for example about 10 um to about 1,000 um, but may also be less, i.e., less than about 10 um, if interactions between or among a plurality of micro-insults are analyzed. Spacing may be on a regular or irregular basis. Preferably, the spacing is on a regular basis, that is, at fixed and uniform intervals.

The micro-insults are monitored using an imaging technique. The imaging technique may be a microscopic or macroscopic imaging technique. The imaging technique is used to evaluate the effects of the micro-insult on changes in the morphology or physiology of the tissue being tested. Monitoring may comprise a single analysis of an area of tissue using a single image, or multiple analyses of an area of tissue using multiple images generated using one imaging technique or more than one imaging technique. For example, monitoring may comprise the analysis or comparison of multiple, sequential images of an area of tissue collected over predetermined time intervals.

Suitable imaging techniques include, but are not limited to confocal microscopy, digital imaging, fluorescence microscopy, optical coherence tomography (OCT), two-photon fluorescence microscopy, second harmonic generation microscopy, coherent anti-Stokes Raman scattering (CARS) microscopy, and spectral imaging. Preferably, the imaging technique is a microscopic imaging technique.

The method of the present invention is advantageous because it is predictive of the effects of macro-insults to the skin (or other tissues), but with minimum adverse effect or damage to the skin due to the extremely small size of the injury. The method provides quick recovery, low discomfort to the test subject, and may be used on different parts of the body, including the sensitive areas of the face and lips, or on internal organs.

As described herein, a particularly suitable use for the present methods is for assessing the efficacy of skin care products. For example, one area of skin may be treated with a sunscreen and another area of skin of the same subject may be untreated. Both areas of skin may then exposed to micro-insults in the form of UV irradiation. Following this, images of both areas may be obtained using an imaging technique and then compared to determine the effect of the sunscreen.

Similarly, the effects of wound healing compositions may be evaluated using the present methods. Micro-insults in the form of puncture wounds may be made to two areas of a subject's skin with microblades. One area may then be treated with a wound healing composition and the other area left untreated. The two areas can then be monitored to determine the effect of the wound healing composition, either by obtaining and comparing single images for each area, or by obtaining and comparing a series of images taken over pre-determined time periods during the healing process.

The methods of the present invention may also be useful for allergy testing. Using small capillary tubes, small amounts of allergens may be applied to an area of the skin to create micro-insults thereon. The skin may be monitored using a microscopic imaging technique for signs of an allergic reaction, such as swelling, erythema and the like.

In one embodiment, the invention is not used for allergy testing.

In one embodiment, the method does not include a step that involves attributing any deviation (from standard values) in the values obtained by the method to a particular clinical picture. In other words, it provides only intermediate values and does not involve a diagnosis for curative purposes *stricto sensu.*

In one embodiment, the method does not involve any substantial health risk, i.e. it is safe to be performed in the absence of a qualified physician.

Examples are set forth below to further illustrate the nature of the invention and the manner of carrying it out. However, the invention should not be considered as being limited to the details thereof.

### Examples

### Example 1

Eight healthy subjects ranging from Fitzpatrick skin type IL-VI with age from 27 to 57 years old were recruited. The volar forearm of each subject was treated with an array of laser beams. Two sites were treated with two different fluences, 40mJ and 60mJ per microscopic wound, respectively. Each site received a single line of micro-exposures over a 1 cm long section of skin at a distance of ∼400 microns from the center of the individual microscopic treatment zones. A thin layer of baby oil was used to ensure coupling between the laser head and the skin surface.

In order to evaluate the short-term and long-term effect of the treatment, the dynamic response of the micro-wound healing process was monitored at 30 minutes after treatment, 2 days, 4 days, and 3 weeks after the treatment *in vivo* using multi-modal microscopic and macroscopic imaging techniques, including video microscopy (KH-300, Hirox, Japan) and reflectance-mode laser scanning confocal microscopy (Vivascan 1500, Lucid, Rochester, NY) with an operating power of less than 20 mW and the lateral resolution of ∼1 micron and the axial resolution of ∼5 microns at 785 nm.

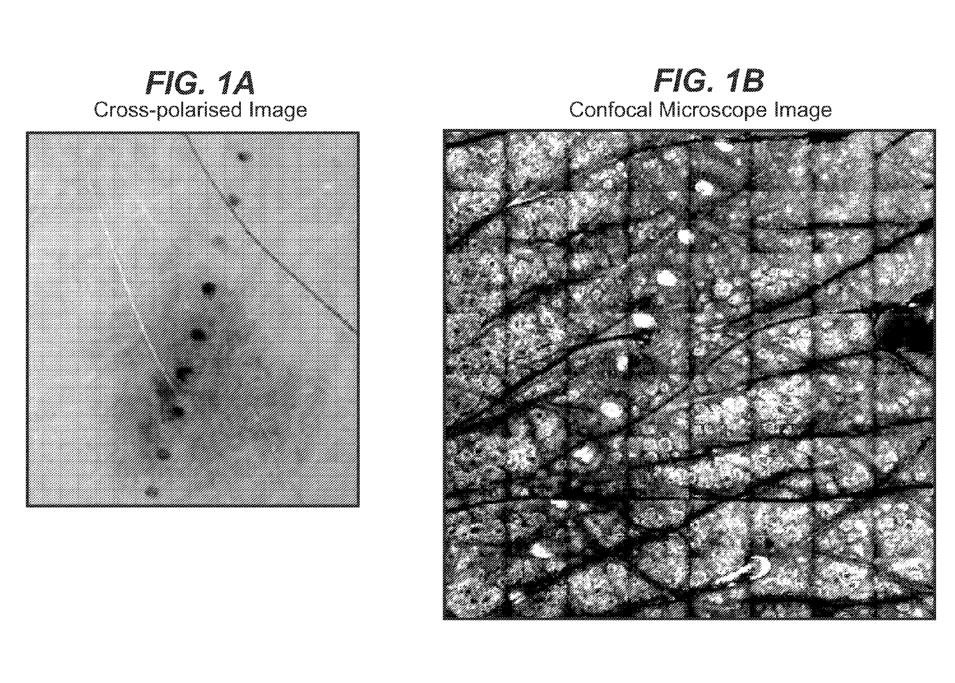

FIGs. 1A and 1B show a cross-polarized image using video microscope and the corresponding reflectance mode confocal microscopic image for one of the subjects. Cross-polarization effectively removed the surface glare and highlighted sub-surface features of pigmentation and vasculature. The micro-wounds appeared as a line of dark brown spots, while their corresponding confocal microscopic image appeared to be bright in the wounded area at around 80 microns below the skin surface, indicating stronger scattering in the micro-wound area.

In order to monitor the wound healing process of the individual micro-wounds and quantify the healing process at the microscopic level, depth-dependent intensity profiles were collected for each microscopic wound, the surrounding collaterally damaged zone and the normal area from confocal microscope images. To minimize the effect of motion during acquisition, images were first co-registered. The intensity profile for each area was obtained using the average intensity within the region of interest at each depth.

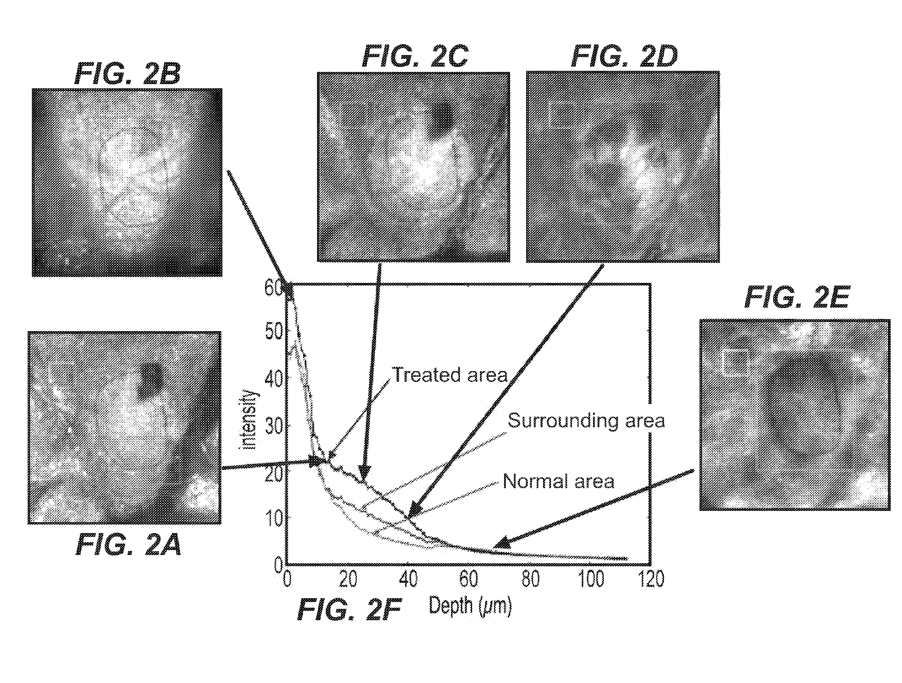

FIGs. 2A, 2B, 2C, 2D, 2D and 2E depict the confocal microscopic images from the volar forearm of a 27-year-old female Caucasian at around 30 minutes after the laser treatment (60 mJ per micro-wound). FIG. 2F shows the corresponding depth intensity profile. At the top 10-15 microns below the skin surface, there was only a slight increase in reflection in the treated microthermal zone compared to the surrounding area and normal area, indicating the minimal damage to the skin at the surface. At 20 to 50 microns below the skin surface, there was a significant increase in reflection within the treated micro-zone. The increased intensity within the micro-zone may have been due to swelling, causing the refractive index mismatch from the superficial layer. From 50 microns and deeper into the dermis, there was a slight decrease in reflection at the treated micro-wound, suggesting that laser treatment may have denatured collagen in the dermis. The depth intensity profile clearly differentiated three areas of interest and provided a quantitative method to evaluate the changes as a response to micro-injury.

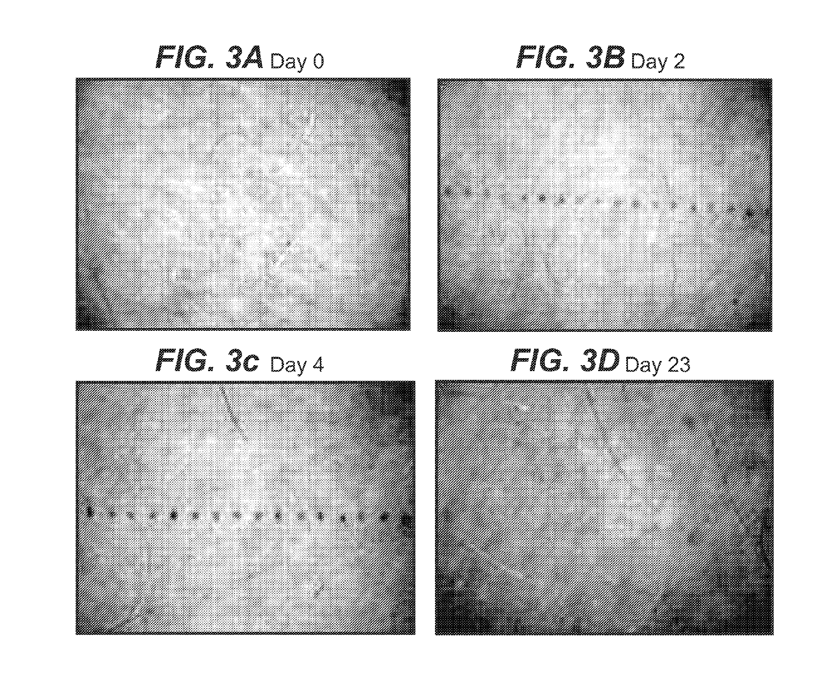

FIGs. 3A, 3B, 3C and 3D show the cross-polarized video microscopic images of a subject as a response to micro-injuries at 60 mJ per microscopic wound at 30 minutes, 2 days, 4 days and 23 days after treatment on the volar forearm, respectively. This enabled an understanding of the dynamic changes of cellular structures and collagen matrix. The micro-wounds were invisible immediately after treatment, with slight edema and inflammation. The micro-zones started to appear as brown-colored micro-wounds within the first 48 hours after treatment. The micro-wounds gradually healed and the wounded micro-zones became invisible after 3 weeks.

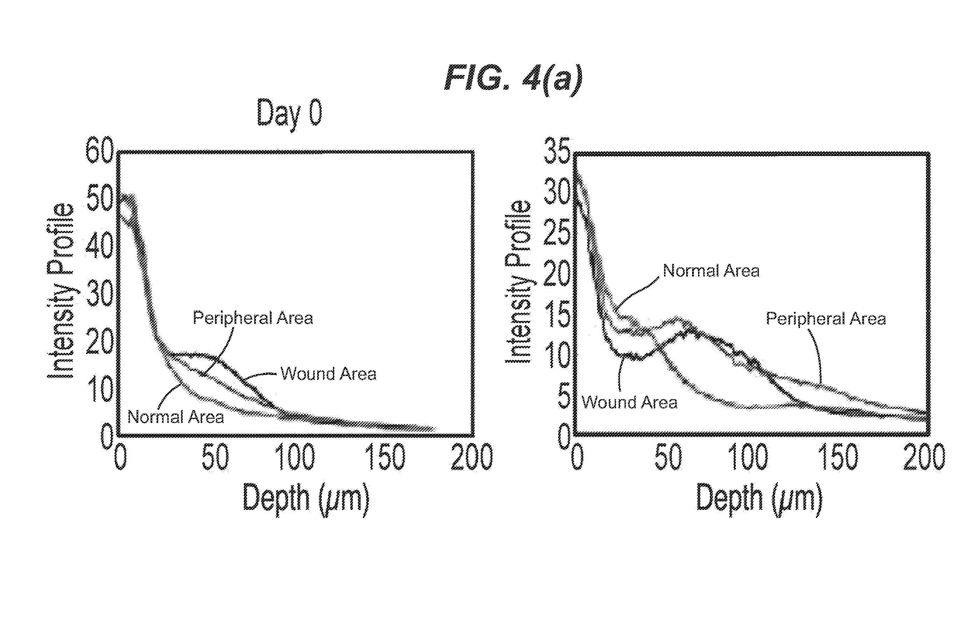

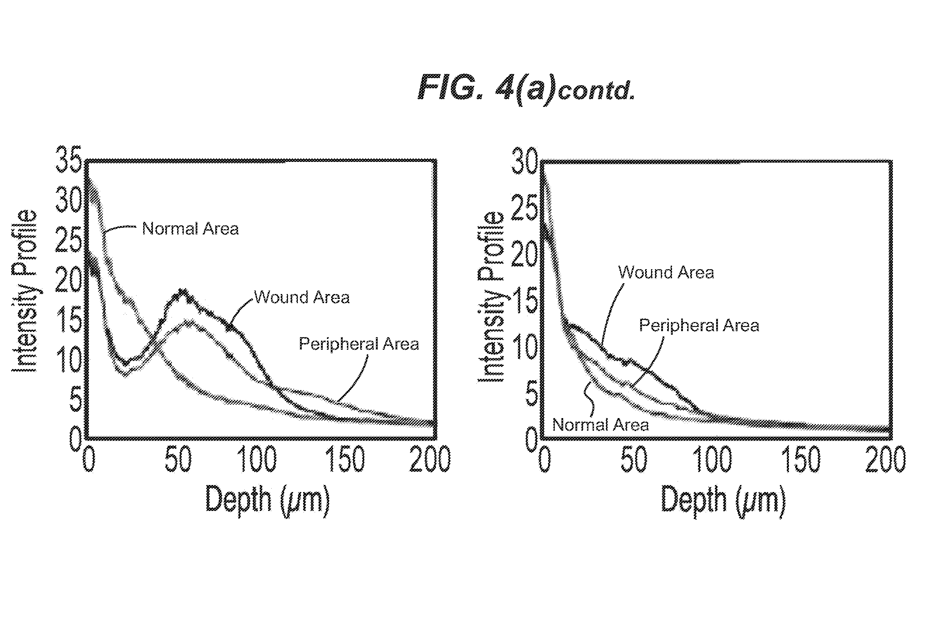

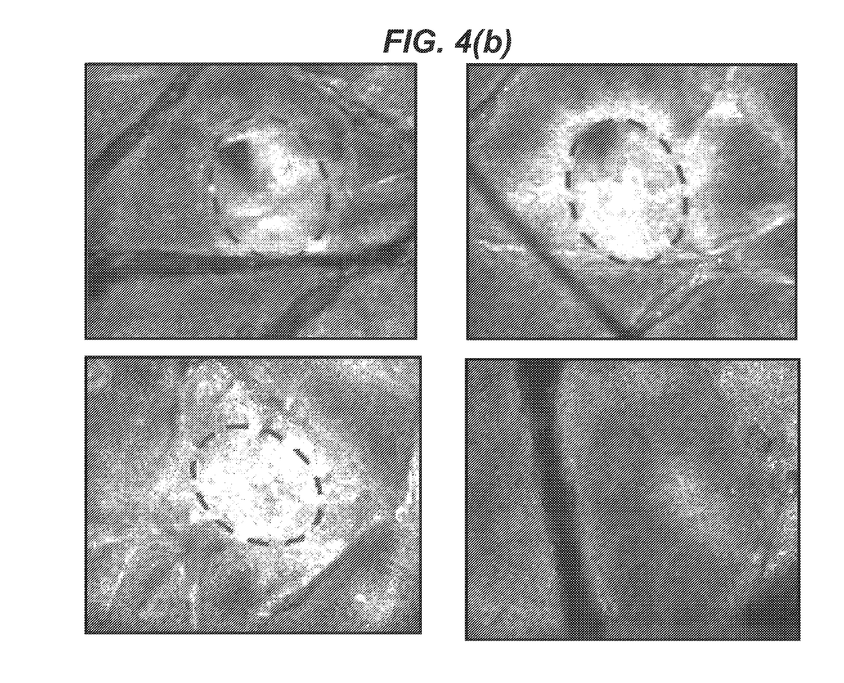

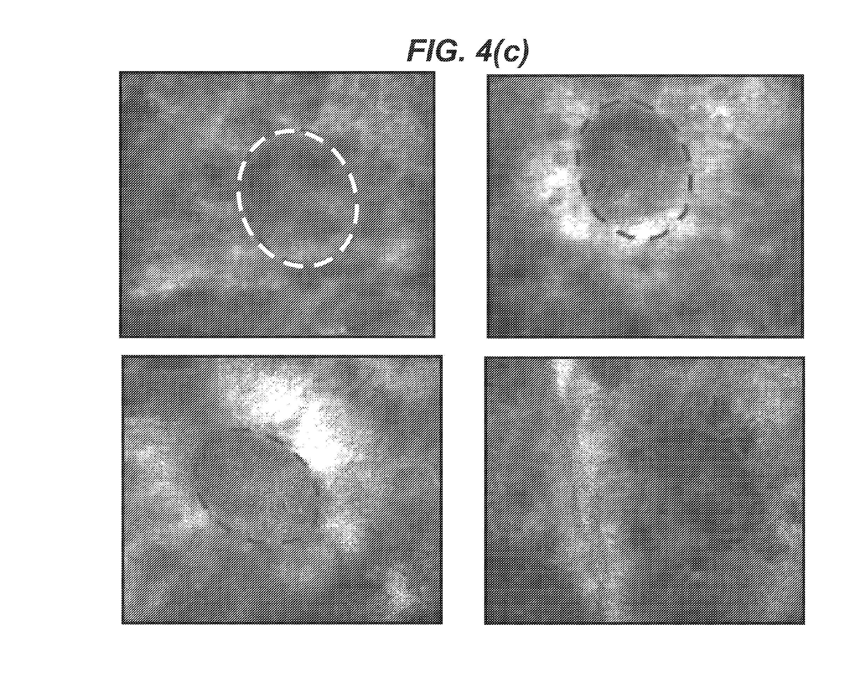

FIG. 4 shows the confocal microscopic images and corresponding depth intensity profiles for the same subject at 30 minutes, 2 days, 4 days and 23 days after treatment. FIG. 4(a) shows the representative depth intensity profile of a treated micro-wound, the surrounding collateral damage zone and normal area at the specified time points. The profile from normal skin retained similar patterns over time. Compared to the normal area, the depth intensity profile of the treated micro-wound showed progressive increase in intensity from 20 to 100 microns below the surface until 4 days after treatment, and then returned to the similar pattern of normal skin after 3 weeks post-treatment. The surrounding collateral damaged zone showed little change compared to normal skin 30 minutes after treatment, then showed a significant increase in intensity after 2 days and 4 days, and then returned to normal after 23 days. The depth intensity profiles also indicated that there was minimal change in the superficial skin within the top 20 microns of the skin surface, confirming the clinical observation of minimal harm to the superficial epidermis. The depth intensity profiles provided a quantitative measure of the associated cellular structure changes.

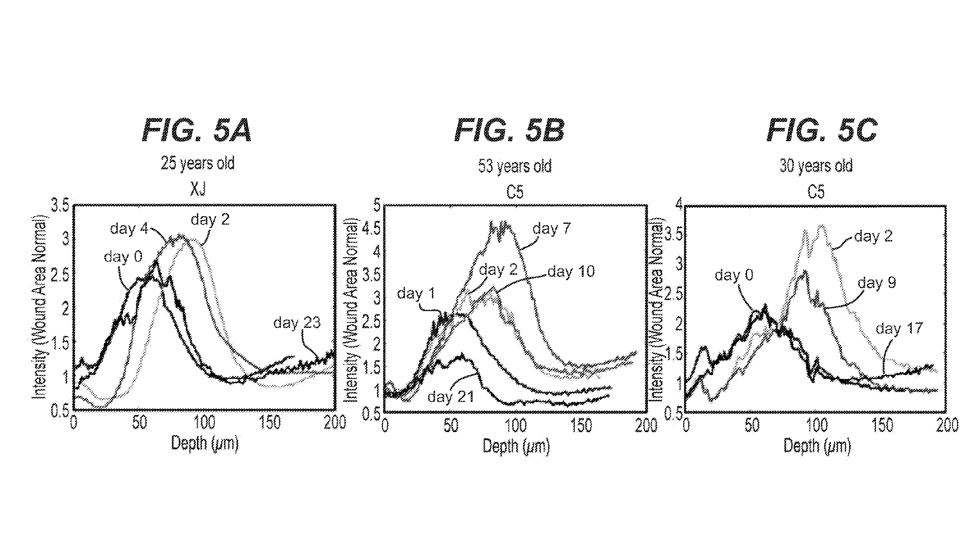

To compensate for the intrinsic exponential decay in intensity associated with increasing depth in confocal microscopy, the depth intensity profiles of the treated micro-injury zones were normalized to that of normal area. FIGs. 5A. 5B, and 5D show the normalized depth intensity profiles of subjects of different ages at different time points after treatment (60 mJ per micro-wound). In general, the normalized depth intensity profiles showed a peak and the peak position first shifted to the deeper depth and then returned to shallower depth. The transition time point when the peak position shifted back to the shallower depth depended on age. For example, for a 25 year old subject, the peak returned to a shallower depth at 4 days after treatment; while for a 30 year old subject, the peak shifted back at 9 days after treatment and for a 53 year old subject, the peak shifted back at 13 days after treatment. This suggests that younger individuals have a faster wound healing rate than older individuals, in agreement with clinical observations. A normalized depth intensity profile may be used as a quantitative method to evaluate the wound-healing rate at a microscopic cellular level.

### Example 2

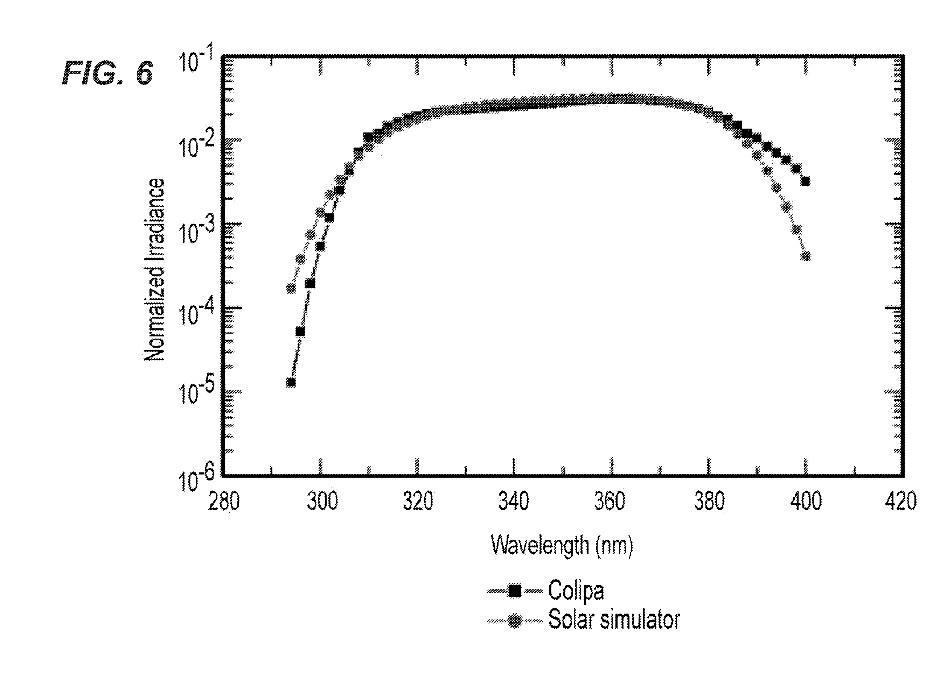

Micro-insults of ultra-violet (UV) radiation from a light source (LightCure 200, Hamamatsu) were delivered to a subject's skin through the use of custom made optical fibers (Multimode) or pinholes (National Aperture, Inc., Salem, NH) of microscopic diameters. Diameters of 50 microns, 200 microns and 500 microns were tested. Subjects were irradiated with solar simulator radiation with irradiance ranging from 20 to 50 mW/cm² and doses varying from 0.5 of the minimum erythema dose (MED) to 3 MED. The light source was filtered with a 1 mm thick UG-11 and a 2 mm thick WG320 (Schott) for solar simulator radiation (280-400 nm). The spectral profile is shown in FIG. 6.

The optical fibers were securely placed onto the skin throughout the experiment so that the irradiation was limited to the diameter of the fiber/slit. Double stick tape was used to attach the fibers to the skin. Multi-modal microscopic and macroscopic imaging techniques such as cross-polarized digital imaging, UV-excited fluorescence imaging, spectral imaging, video microscopy (KH-300, Hirox, Japan) and reflectance-mode laser scanning confocal microscopy (Vivascan 1500, Lucid, Rochester, NY), were used to evaluate the progress of healing after irradiation. In order to monitor the micro-insults, the optical probes/slits were arranged in a line.

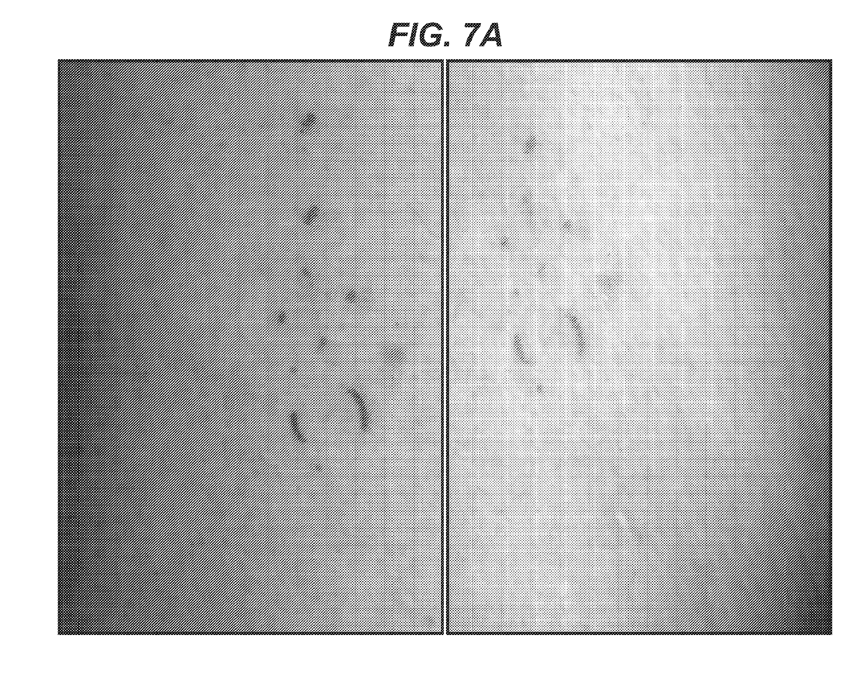

FIG. 7A shows cross-polarized images of irradiated sites taken 24 hours (right) and 48 hours (left) after solar simulator exposure and show skin erythema. The sites between the parenthesis and four dots received irradiations with a line of 200 um diameter fibers at 152.5 mJ/cm² and 254.1 mJ/cm², respectively. The site within the two dots received irradiation through a 0.5 mm slit at 24.4 mJ/cm².

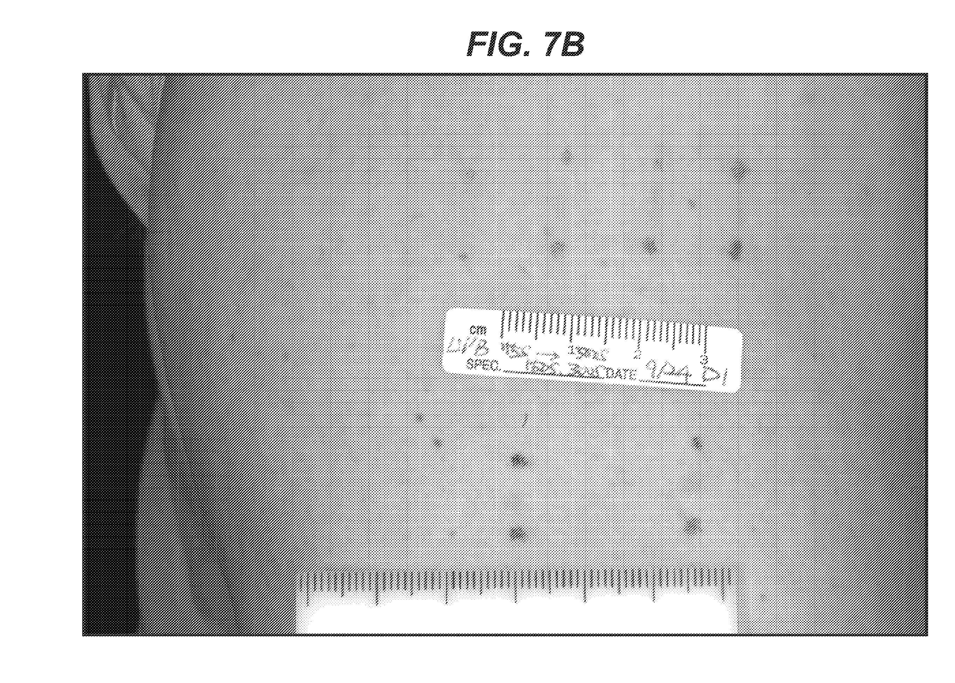

FIG 7B shows the erythema level for irradiations with the 200 um optical fiber probe (top) and the 2 mm diameter circular optical fiber probe (bottom). The doses for the 200 um optical fiber probe were 60, 120, 240 and 400 mJ/cm² from left to right, and 120 and 240 mJ/cm² for the 2 mm diameter circular optical fiber probe.

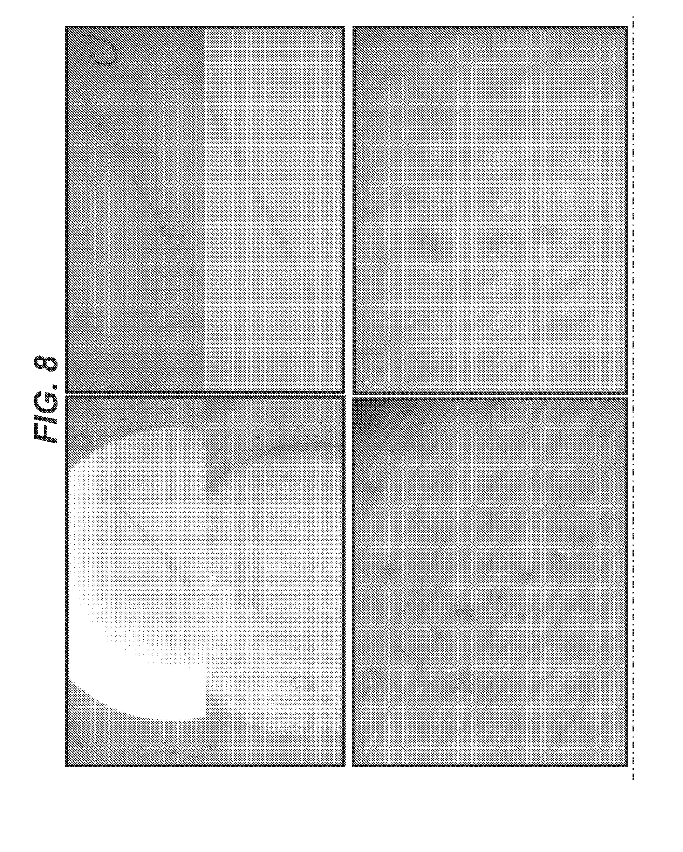

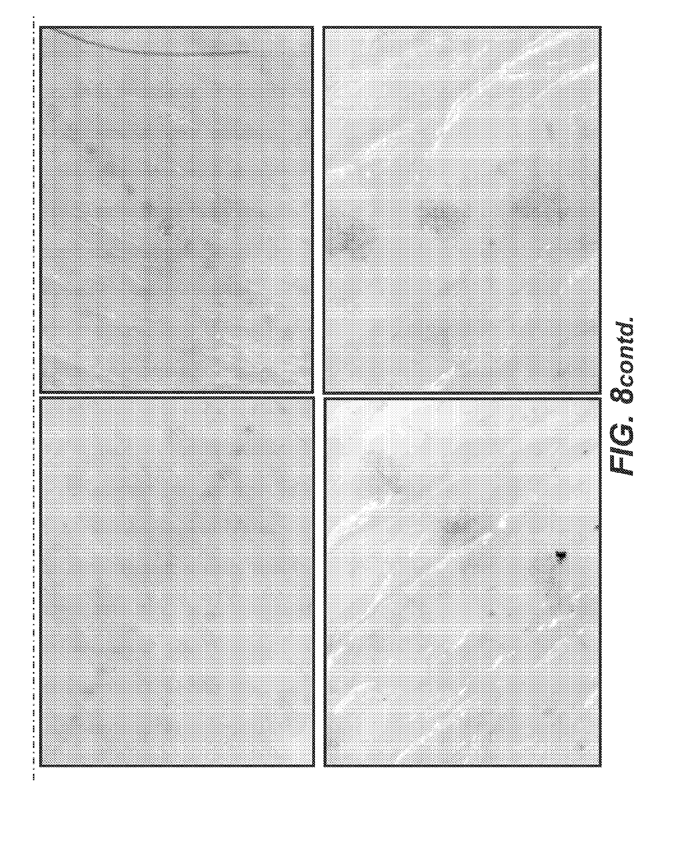

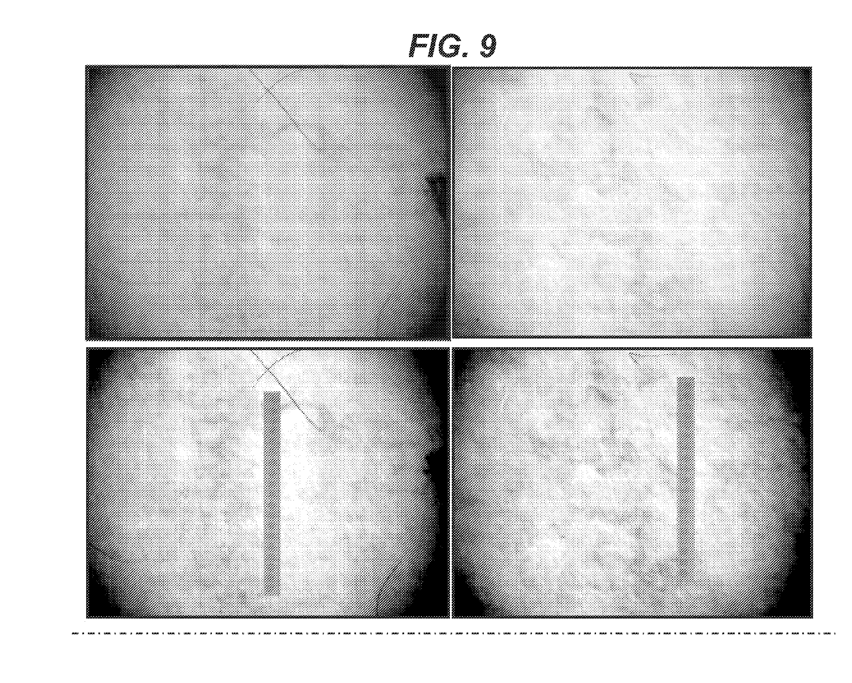

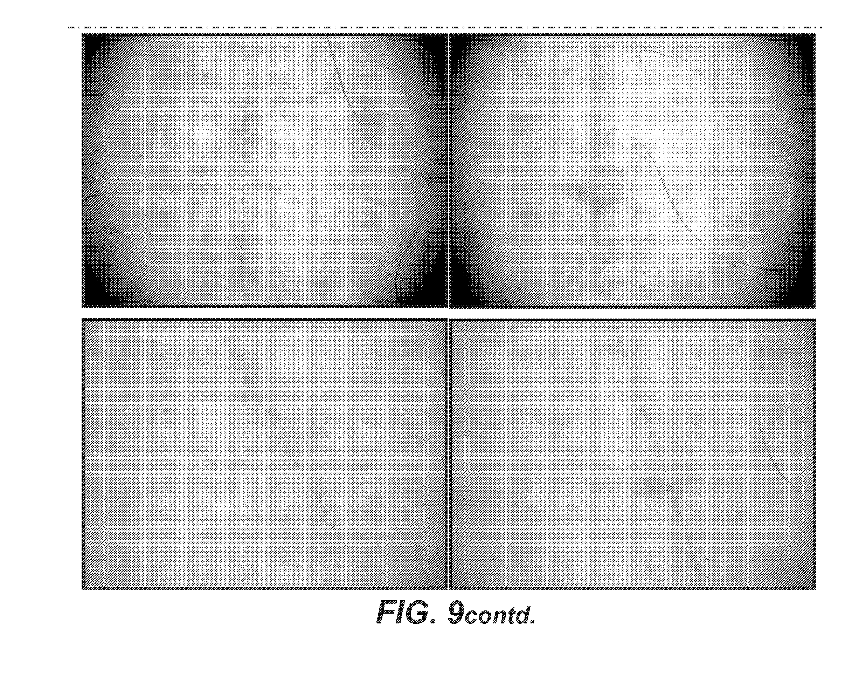

Microscopic imaging (HiScope) taken at different days after skin irradiation was used to track the development of the micro-injuries over an extended period of time at several optical magnifications and is shown in FIG. 8. FIG. 9 shows similar images taken at sequential timepoints after irradiation. The different aspects of the micro-injuries such as erythema immediately and at 24 hours and pigmentation at 6 and 11 days post irradiation are depicted.

### Example 3

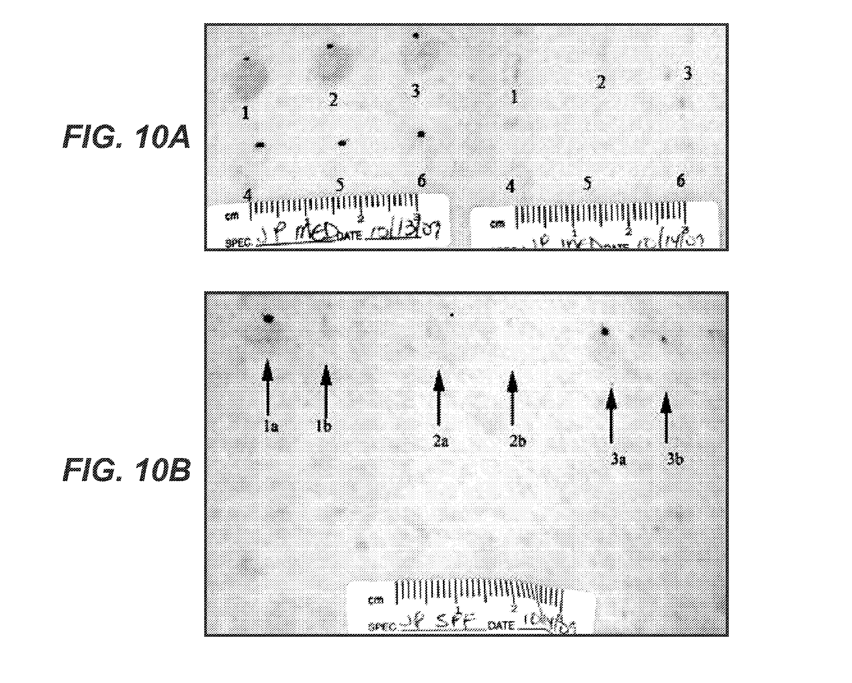

The performance of a sunscreen with sun protection factor SPF 48 was evaluated *in vivo* according to the invention as follows. A solar simulator of the same type described in Example 2 was used to apply microscopic insults of UV radiation in increments of 25%. First, the skin was tested without sunscreen to determine the MED for the subject. FIG. 10A shows the results. This test was done with both a micro-injury fiber and a regular phototest fiber having an 8 mm diameter for comparison as recommended by the COLIPA standard. Doses of 15.5, 12.4, 9.9, 7.9, 6.3, and 5.1 mJ/cm² for sites 1-6, respectively, were applied. The MED was determined to be 9.9 mJ/cm² (site 3).

The sunscreen was then uniformly applied (2 ml/mm²) and the skin was irradiated with doses 470, 381, 304.8, mJ/cm2 for sites 1-3, respectively (FIG. 10B). Sites "1a," "2a," and "3a" were irradiated using an 8 mm diameter fiber and sites "1b," "2b" and "3b" were irradiated using a micro-injury fiber. The MED with sunscreen was determined to be 470 mJ/cm2 (site 1). The SPF *in vivo* may be calculated as 470/9.9 = 47.5, which correlates very well with the nominal value of 48.

As demonstrated by this Example, method of the invention may be used to predict the *in vivo* SPF value of a sunscreen composition accurately.

Embodiments of the invention are set out in the following numbered clauses.
1. A method for testing human skin, which comprises:
   causing at least one micro-insult to an area of the human skin; and
   monitoring the area using an imaging technique.
2. The method of clause 1, wherein the imaging technique is a microscopic imaging technique.
3. The method of clause 1, wherein the imaging technique is selected from the group consisting of to confocal microscopy, digital imaging, fluorescence microscopy, optical coherence tomography, two-photon fluorescence microscopy, second harmonic generation microscopy, coherent anti-Stokes Raman scattering microscopy, and spectral imaging.
4. The method of clause 1, wherein the micro-insult has a maximum dimension of less than about 500 microns.
5. The method of clause 1, wherein at least two micro-insults spaced apart by at least 10 microns are applied to the area.
6. The method of clause 1, wherein the micro-insult is caused by an action selected from the group consisting of punctures, cuts, burns, irradiation, and exposure to allergens.
7. A method for determining the effects of a topically applied composition to human skin, which comprises in sequence:
   applying the composition to an area of the human skin;
   causing at least one micro-insult to the area; and
   monitoring the area using an imaging technique.
8. The method of clause 7, wherein the imaging technique is selected from the group consisting of to confocal microscopy, digital imaging, fluorescence microscopy, optical coherence tomography, two-photon fluorescence microscopy, second harmonic generation microscopy, coherent anti-Stokes Raman scattering microscopy, and spectral imaging.
9. The method of clause 7, wherein the micro-insult has a maximum dimension of less than about 500 microns.
10. The method of clause 7, wherein at least two micro-insults spaced apart by at least 10 microns are applied to the area.
11. The method of clause 7, wherein the micro-insult is caused by an action selected from the group consisting of punctures, cuts, burns, irradiation, and exposure to allergens.
12. A method for determining the effects of a topically applied composition to human skin, which comprises in sequence:
   causing at least one micro-insult to an area of the human skin;
   applying the composition to the area; and
   monitoring the area using an imagining technique.
13. The method of clause 12, wherein the imaging technique is selected from the group consisting of to confocal microscopy, digital imaging, fluorescence microscopy, optical coherence tomography, two-photon fluorescence microscopy, second harmonic generation microscopy, coherent anti-Stokes Raman scattering microscopy, and spectral imaging.
14. The method of clause 12, wherein the micro-insult has a maximum dimension of less than about 500 microns.
15. The method of clause 12, wherein at least two micro-insults spaced apart by at least 10 microns are applied to the area.
16. The method of clause 12, wherein the micro-insult is caused by an action selected from the group consisting of punctures, cuts, burns, irradiation, and exposure to allergens.

## Claims

1. A method for testing human skin, which comprises:
causing at least one micro-insult to an area of the human skin; and
monitoring the area using an imaging technique.

2. A method of claim 1 for determining the effects of a topically applied composition to human skin, which comprises in sequence:
applying the composition to an area of the human skin;
causing at least one micro-insult to the area; and
monitoring the area using an imaging technique.

3. A method of claim 1 for determining the effects of a topically applied composition to human skin, which comprises in sequence:
causing at least one micro-insult to an area of the human skin;
applying the composition to the area; and
monitoring the area using an imagining technique.

4. The method of any one of the preceding claims, wherein the imaging technique is a microscopic imaging technique.

5. The method of any one of the preceding claims, wherein the imaging technique is selected from the group consisting of confocal microscopy, digital imaging, fluorescence microscopy, optical coherence tomography, two-photon fluorescence microscopy, second harmonic generation microscopy, coherent anti-Stokes Raman scattering microscopy, and spectral imaging.

6. The method of any one of the preceding claims, wherein the micro-insult has a maximum dimension of less than about 500 microns.

7. The method of any one of the preceding claims, wherein at least two micro-insults spaced apart by at least 10 microns are applied to the area.

8. The method of any one of the preceding claims, wherein the micro-insult is caused by an action selected from the group consisting of punctures, cuts, burns, irradiation, and exposure to allergens.
